(19) Europäisches Patentamt
European Patent Office
Office européen des brevets

(11) **EP 1 060 715 A1**

(12) **EUROPEAN PATENT APPLICATION**

(43) Date of publication:
20.12.2000 Bulletin 2000/51

(51) Int. Cl.⁷: **A61F 2/14**

(21) Application number: **00115411.1**

(22) Date of filing: **04.03.1996**

(84) Designated Contracting States:
**AT BE CH DE DK ES FI FR GB GR IE IT LI LU MC NL PT SE**

(30) Priority: **02.03.1995 US 399300**
**12.05.1995 US 437415**

(62) Document number(s) of the earlier application(s) in accordance with Art. 76 EPC:
**96911252.3 / 0 820 260**

(71) Applicant: **KERAVISION, INC.**
**Fremont, California 94538-7353 (US)**

(72) Inventors:
• **Silvestrini, Thomas A.**
**Alamo, California 94507 (US)**

• **Mathis, Mark L.**
**Fremont, California 94530 (US)**
• **Scholl, John A.**
**Danville, California 94506 (US)**
• **Proudfoot, Robert A.**
**Santa Clara, California 95051 (US)**

(74) Representative:
**Price, Nigel John King**
**J.A. KEMP & CO.**
**14 South Square**
**Gray's Inn**
**London WC1R 5LX (GB)**

Remarks:
This application was filed on 17 - 07 - 2000 as a divisional application to the application mentioned under INID code 62.

(54) **Corneal implant for changing refractive properties**

(57) An intrastromal corneal ring for insertion into the eye of a cornea in order to improve the vision of the eye. The ring comprises a biocompatible ring and has multiple cone angles, such that the cone angle is differ-ent at differerent circumferential positions on the ring.

FIG. 13

**Description**

Technical Field

The present invention relates to a device and method for changing corneal refractive properties including the radius of curvature and/or the aspheric shape of the cornea of an eye. More specifically, the invention involves an intrastromal corneal ring having a cone angle or multiple cone angles which effect this change when the intrastromal corneal ring is inserted into the cornea, and the method for effecting that change.

Background

Anomalies in the shape of the eye can cause visual disorders. Axial hyperopia ("farsightedness") occurs when the front-to-back distance in the eyeball is too small. Curvature hyperopia occurs when the corneal curvature is less than normal and therefore is flatter than the normal cornea. In these cases, parallel rays originating greater than 20 feet from the eye focus behind the retina. In contrast, when the front-to-back distance of the eyeball is too large, axial myopia ("nearsightedness") occurs. When the corneal curvature is too great, curvature myopia occurs. In these cases, the focus of parallel rays entering the eye occurs in front of the retina. Astigmatism is a condition which occurs when the parallel rays of light do not focus to a single point within the eye, but rather have a variable focus due to the fact that the corneal curvature varies in different meridians. Light is therefore refracted different distances and focuses at different regions. Some degree of astigmatism is normal, but where astigmatism is too pronounced, it must often be corrected. Presbyopia is an age-related condition that results in the loss of the ability of the eye to change focal length.

Hyperopia, myopia, presbyopia and astigmatism are usually corrected by glasses or contact lenses. Surgical methods for the correction of such disorders have been cited in the literature and include radial keratotomy (see e.g. U.S. Patents Nos. 4,815,463 and 4,688,570) and laser corneal ablation (see e.g. U.S. Patent No. 4,941,093). Another method for correcting those disorders is through implantation of polymeric rings in the eye's corneal stroma to change the curvature of the cornea. Previous work involving the implantation of achier (2) rings, allograft corneal tissue and hydrogels is well documented. One of the ring devices involves a ring design that allows a split ring to be inserted into a channel dissected in the stromal layer of the cornea. The device uses a minimally invasive incision through which the channel for the implant is created and through which the implant is inserted and adjusted. Adjustment of the device normally involves an adjustment of ring size or diameter.

U.S. Patent No. 4,452,235 describes a method and apparatus for corneal curvature adjustment. The method involves inserting one end of a split end adjusting ring into the cornea of the eye and moving the ring in a circular path until its ends meet. The ends are thereafter adjusted relative to each other until the shape of the eye has assumed a desired curvature whereupon the ends are fixedly attached to maintain the desired curvature of the cornea.

PCT/US93/00059, the entirety of which is incorporated by reference, describes a method for the refractive correction of the eye as well. Intrastromal corneal rings of varying thickness are inserted into the corneal stroma to change the curvature of the cornea.

The present invention involves the use of intrastromal corneal rings of varying cone angles to change the curvature of the cornea for the refractive adjustment of the eye.

SUMMARY OF THE INVENTION

The present invention involves changing the configuration of the cornea as a function of cone angle. According to the present invention, an intrastromal corneal ring is provided with a mismatching cone angle selected to independently impart a force on the corneal tissue when the intrastromal corneal ring is positioned at the desired location in the cornea. More specifically, the mismatching cone angle can independently effect a change in the radius of curvature and/or the aspheric shape of the cornea. Thus, the cone angle is chosen based on the starting curvature of the eye, the thickness of the intrastromal corneal ring and the type of corneal curvature and/or aspheric change desired. The cone angle may be selected to (1) maintain the surface of the eye close to aspheric shape of the eye prior to insertion of the ring or (2) alter the aspheric shape of the eye as desired, for example.

According to the present invention, a mismatching angle preferably is described with reference to an imaginary intrastromal conical ring superimposed on the insertion site prior to insertion. This permits calculation of the appropriate angle with reference to the cornea before its configuration is changed through the insertion of the intrastromal corneal ring. Returning to the description, the major axis of substantially any radial, transverse cross-section of the intrastromal cortical ring would not be parallel to a line in the same plane as said axis and tangent to the anterior surface of the cornea at the point where the line that bisects said major axis line (defined as the line extending along said major axis and bounded by the outer surface of the intrastromal corneal ring and is perpendicular thereto) intersects the anterior surface of the cornea.

Mismatching cone angles for different corneal radius of curvatures also can be described as those that fall outside the range of matching cone angles described according to the following equations:

$$\theta = \sin^{-1}\left[\frac{D_{cc}}{2R_i}\right], \text{ where}$$

$\theta$ = cone angle
$D_{cc}$ = diameter of the intrastromal corneal ring (center to center)
$R_i$ = initial corneal radius of curvature

The foregoing equation can be rewritten to account for implanting the conical ring at a depth "d". The following equation is the same as the foregoing with the exception that "d" is zero in the foregoing equation.

$$\theta = \sin^{-1}\left[\frac{D_{cc}}{2(R_i-d)}\right], \text{ where}$$

d = depth of the intrastromal corneal ring in the cornea measured radially from the anterior corneal surface to the midpoint of a radial line, extending across the thickest or largest radial dimension of the above-referenced radial, transverse section of the intrastromal corneal ring and bounded thereby.

Alternate equations may be used to account for intrastromal corneal ring thickness when intrastromal corneal rings with relatively large thicknesses are used. This is generally preferred when accounting for the thicknesses above about 0.15 mm (i.e., thicknesses that provides sufficient thickness to flatten the cornea independent of cone angle). According to this refined equation:

$$\theta = \sin^{-1}\left[\frac{D_{cc}}{2(R_i-\Delta R_i)}\right]$$

$D_{cc}$ = diameter of the intrastromal corneal ring (center to center)
$R_i$ = initial corneal radius of curvature
$\Delta R_t$ = the expected change induced by intrastromal corneal ring thickness.

Again, when accounting for "d", the foregoing equation can be refined to the following:

$$\theta = \sin^{-1}\left[\frac{D_{cc}}{2[(R_i-d)+|\Delta R_t|]}\right]$$

According to a preferred method for changing the refractive characteristics of an eye, the method comprises the steps of: (a) providing a group of intrastromal corneal rings having different cone angles; (b) determining an amount of corrective refraction desired; (c) selecting an intrastromal corneal ring from the group of intrastromal corneal rings based on the amount of corrective refraction determined in step (b); and (d)inserting the intrastromal corneal ring selected in step (c) into the cornea of the eye.
With this method, the corneal curvature can be changed by using intrastromal corneal rings with different cone angles. In addition, if a desired amount of refractive correction has not been achieved (the refractive correction can be measured after step (d)), the inserted intrastromal corneal ring can be removed and a second intrastromal corneal ring from the group and having a different cone angle implanted. More specifically, the second intrastromal corneal ring can be selected to have a cone angle greater than the cone angle of the intrastromal corneal ring implanted in step (c) if the eye or cornea does not flatten (e.g., from center to periphery) by the desired amount to treat myopia, for example. According to another aspect of this method, the second intrastromal corneal ring can be selected to have a cone angle less than the cone angle of the intrastromal corneal ring implanted in step (c) if the region of the cornea inside the intrastromal corneal ring does not steepen by the desired amount. This generally would be the case when treating a hyperopic condition.
In addition, a number of the intrastromal corneal rings provided in step (a) can be provided with different thicknesses and/or diameters to accommodate a number of different corneal configurations. For example, a number of the intrastromal corneal rings can have the same outer diameter but with different cone angles. In this manner, a suitable mismatching cone angle can be selected to effect a desired change in corneal shape as described above.

According to a further embodiment of the invention, an intrastromal corneal ring comprising a biocompatible ring having multiple cone angles is provided. That is, the cone angle changes along the circumferential direction of the intrastromal corneal ring. This construction is particularly advantageous for treating astigmatism (or astigmatism concurrent with either myopia or hyperopia) where the corneal curvature varies in different meridians.

According to another emobidment of the invention, a kit of rings containing a number of different cone angles to choose from is provided. Typically, a kit having multiple rings, preferably with the same $D_{cc}$, but different cone angles will be provided for clinical use. These rings will typically vary from a matched cone angle for a given $R_i$ by at least 1°, more preferably by at least 2°, even more preferably by at least 3° and yet more preferably by at least 5° (to facilitate substantial corrections) so that if one does not provide the desired correction, another one can be used (i.e., if the first ring selected does not provide the desired correction, it can be replaced by another one from the kit). Preferably, the kit will include a ring having a cone angle greater than and a ring having a cone angle less than that of a matched cone angle for a given $R_i$ by the foregoing amounts to facilitate flattening or steepening of the cornea. The kit also may include other rings, including one having a matching cone angle. All or a lesser number of the rings also may vary in 0.5° (or larger) increments from one another and have the same thickness. With a kit having only rings with the same $D_{cc}$ or ring diameter, selection of the properly mismatching ring may be simplified. This feature also allows the kit to be made as small as possible, while covering as large a range of correction as possible.

The above is a brief description of some of the advantages of the present invention. Other features, advantages and embodiments of the invention will be apparent to those skilled in the art from the following description, accompanying drawings and appending claims.

BRIEF DESCRIPTION OF THE DRAWINGS

Fig. 1 is a schematic representation of a horizontal section of the eye.

Fig. 2 is a schematic illustration of the anterior portion of the eye showing the various layers of the cornea.

Fig. 3 is a schematic representation of an eye showing the average corneal curvature radius and aspheric shape of the cornea.

Fig. 4 is a schematic representation of a hyperopic eye showing the average corneal curvature radius and the aspheric shape of the cornea.

Fig. 5 is a plan view of an intrastromal corneal ring according to the present invention.

Fig. 6A is a radial, transverse cross-sectional view of the intrastromal corneal ring of Fig. 5.

Fig. 6B is a further illustration of the corneal ring of Fig. 6A depicting further ring geometry.

Fig. 7A is a cross-sectional view of a cornea showing an intrastromal corneal ring having a cross-sectional configuration the same as the intrastromal corneal ring of Fig. 6A and 6B and a matching cone angle.

Fig. 7B is a diagrammatic view of the intrastromal ring of Fig. 7A illustrating geometric relationships relative to the cornea.

Fig. 8 is a cross-sectional view of a cornea showing an intrastromal corneal ring having a cross-sectional configuration as in Fig. 7 but with an active or mismatching cone angle that according to one embodiment of the invention effects the flattening of the cornea for treating myopia.

Fig. 9 is a cross-sectional view of a cornea showing an intrastromal corneal ring having a cross-sectional configurations in Fig. 7 but with an active or mismatching cone angle that according to another embodiment of the invention effects the steepening of the corneal anterior surface for treating hyperopia.

Figs. 10 and 11 illustrate geometric relationships between an active or mismatching intrastromal corneal ring and a cornea in accordance with the present invention.

Figs. 12A, 12B 12C, 12D and 12E are graphs showing the relationship among intrastromal corneal ring stiffness, correction in diopters and cone angle for a given intrastromal corneal ring and two different stiffnesses.

Fig. 13 illustrates a further embodiment of the intrastromal corneal ring of the present invention in which the intrastromal corneal ring is provided with different cone angles.

Fig. 14 is a sectional view taken along line 14-14 in Fig. 13.

Fig. 15 is a sectional view taken along line 15-15 in Fig. 13.

Like elements in the drawings bear the same reference numerals.

MODES FOR CARRYING OUT THE INVENTION

Prior to explaining the details of the inventive devices and methods, a short explanation of the physiology of the eye is needed to appreciate the functional relationship of the device to the eye.

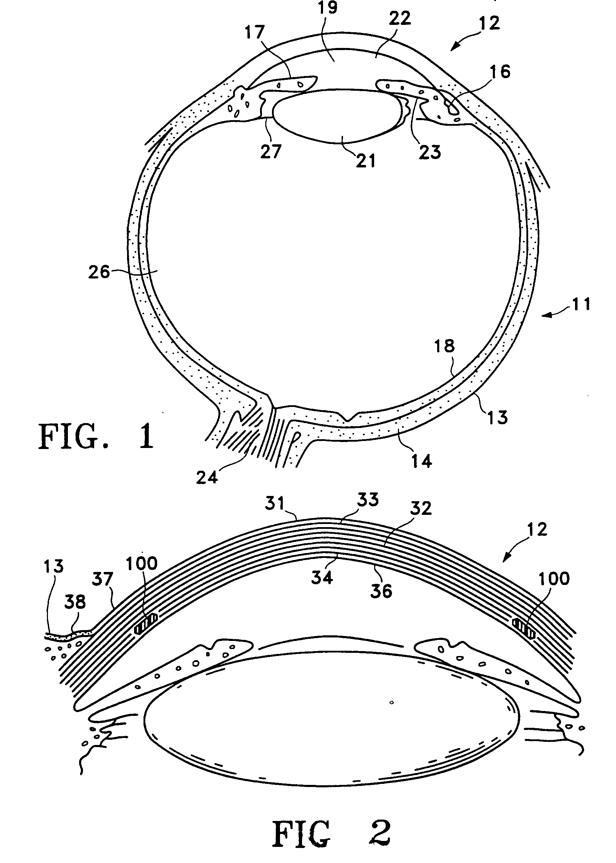

Fig. 1 shows a horizontal section of the eye with the globe (11) of the eye resembling a sphere with an anterior bulged spherical portion representing the cornea (12).

The globe (11) of the eye consists of three concentric coverings enclosing the various transparent media through which the light must pass before reaching the sensitive retina (18). The outermost covering is a fibrous protective portion the posterior five-sixths of which is white and opaque and called the sclera (13), and sometimes referred to as the white of the eye where visible to the front. The anterior one-sixth of this outer layer is the transparent cornea (12).

A middle covering is mainly vascular and nutritive in function and is comprised of the choroid (14), ciliary body (16) and iris (17). The choroid (14) generally functions to maintain the retina (18). The ciliary body (16) is involved in suspending the lens (21) and accommodation of the lens. The iris (17) is the most anterior portion of the middle covering of the eye and is arranged in a frontal plane. It is a thin circular disc corresponding to the diaphragm of a camera, and is perforated near its center by a circular aperture called the pupil (19). The size of the pupil varies to regulate the amount of light which reaches the retina (18). It contracts also to accommodation, which serves to sharpen the focus by diminishing spherical aberration. The iris (17) divides the space between the cornea (12) and the lens (21) into an anterior chamber (22) and posterior chamber (23). The innermost portion of covering is the retina (18), consisting of nerve elements which form the true receptive portion for visual impressions.

The retina (18) is a part of the brain arising as an outgrowth from the fore-brain, with the optic nerve (24) serving as a fiber tract connecting the retina part of the brain with the fore-brain. A layer of rods and cones, lying just beneath a pigmented epithelium on the anterior wall of the retina serve as visual cells or photoreceptors which transform physical energy (light) into nerve impulses.

The vitreous body (26) is a transparent gelatinous mass which fills the posterior four-fifths of the globe (11). At its sides it supports the ciliary body (16) and the retina (18). A frontal saucer-shaped depression houses the lens.

The lens (21) of the eye is a transparent bi-convex body of crystalline appearance placed between the iris (17) and vitreous body (26). Its axial diameter varies markedly with accommodation. A ciliary zonule (27), consisting of transparent fibers passing between the ciliary body (16) and lens (21) serves to hold the lens (21) in position and enables the ciliary muscle to act on it.

Referring again to the cornea (12), this outermost fibrous transparent coating resembles a watch glass. Its curvature is somewhat greater than the rest of the globe and is ideally spherical in nature. However, often it is more curved in one meridian than another giving rise to astigmatism. A central third of the cornea is called the optical zone with a slight flattening taking place outwardly thereof as the cornea thickens towards its periphery. Most of the refraction of the eye takes place through the cornea.

Referring to Fig. 2, a more detailed drawing of the anterior portion of the globe shows the various layers of the cornea (12) comprising an epithelium (31). Epithelial cells on the surface thereof function to maintain transparency of the cornea (12). These epithelial cells are rich in glycogen. enzymes and acetylcholine and their activity regulates the corneal corpuscles and controls the transport of water and electrolytes through the lamellae of the stroma (32) of the cornea (12).

An anterior limiting lamina (33), referred to as Bowman's membrane or layer, is positioned between the epithelium (31) and the stroma (32) of the cornea. The stroma (32) is comprised of lamella having bands of fibrils parallel to each other and crossing the whole of the cornea. While most of the fibrous bands are parallel to the surface, some are oblique, especially anteriorly. A posterior limiting lamina (34) is referred to as Descemet's membrane. It is a strong membrane sharply defined from the stroma (32) and resistant to pathological processes of the cornea.

The endothelium (36) is the most posterior layer of the cornea and consists of a single layer of cells. The limbus (37) is the transition zone between the conjunctiva (38) and sclera (13) on the one hand and the cornea (12) on the other.

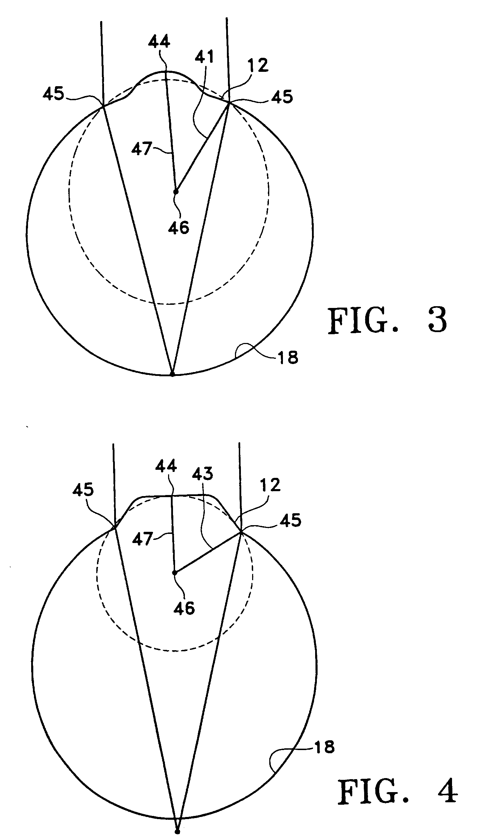

Fig. 3 shows the globe of the eye having a cornea (12) with an average spherical radius of curvature (41) and a positive aspheric shape. By "average spherical radius of curvature" we intend the radius of the circle defined by the points at the periphery (45) of the cornea near the limbus of the eye and having a center (46). By "positive aspheric shape" we mean that the distance (47) from that center (46) to the anterior center of the cornea is greater than the average spherical radius of curvature, that is, the anterior surface of the cornea flattens as it progresses from the center (44) to its periphery (45). As shown in Fig. 3, when parallel rays of light pass through the corneal surface, they are refracted by the corneal surfaces to converge eventually near the retina (18) of the eye. The diagram of Fig. 3 discounts, for the purposes of this discussion, the refractive effect of the lens or other portions of the eye.

The eye depicted in Fig. 4 is hyperopic because the light rays from the periphery of the cornea refract into focus at a point behind the retinal surface. Further, the eye depicted in Fig. 4 has does not have the same aspheric shape as that shown in Fig. 3. The distance (47) from the center (46) to the anterior surface of the cornea is about the same as or less than the average spherical radius of curvature (41) and the cornea does not flatten from center (44) to periphery (45) but rather plateaus or even dips at its center. If an intrastromal conical ring with a flat mismatched cone angle, according to the present invention and as will be described in detail below, is implanted into the cornea shown in Fig. 4, the light rays refracted by the now steepened corneal surface will be refracted at a larger angle and thus converge at a more near point such as directly on the retina. Further, selection of an intrastromal corneal ring having a mismatched cone angle may allow for the eye to obtain a more positive aspheric shape similar to that shown in the Fig. 3 eye.

With the background discussion of Figs. 1-4 in hand, it should be understood that the device and method of the present invention is for the adjustment of at least a portion of an annular chord of the cornea to decrease (or increase) the radius of curvature and/or change the shape of the cornea in order to improve the vision of the eye. According to the present invention, the corneal geometry or refractive properties of the cornea are changed as a function of cone angle which is described in detail below.

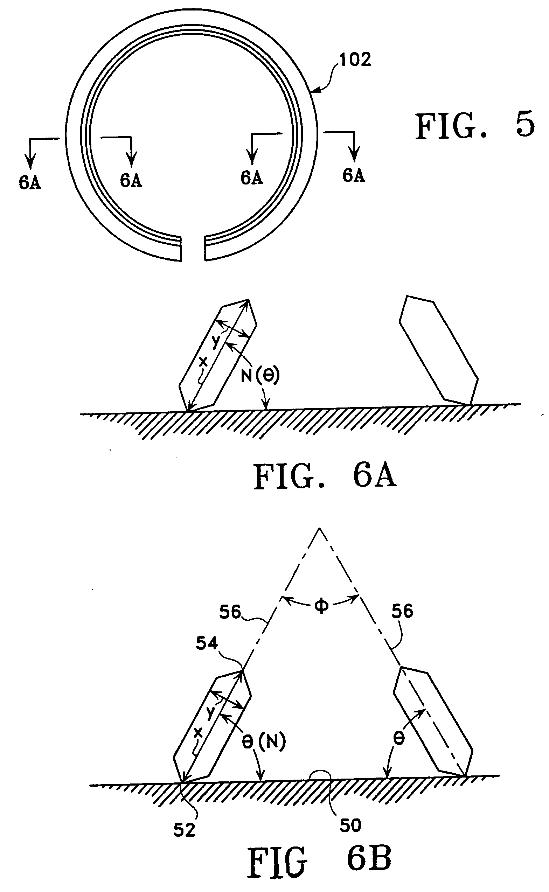

Referring to Fig. 6A, the cone angle "N" (referred to as θ in the equations that follow) of an intrastromal corneal ring is defined as the angle between the plane of the flat surface that the intrastromal corneal ring rests on and a line drawn betwen the point of the cross-section that rests on the flat surface and the point on the cross-section that is farthest from the point where the intrastromal corneal ring rests on the flat surface. The cross-section referred to is one that cuts through the diameter of the intrastromal corneal ring and is at an angle of 90° to the flat surface.

Fig. 5 shows one desirable intrastromal corneal ring made according to the invention. The intrastromal conical ring is comprised of a generally circular member having split end portions. The material should have properties that render it physiologically compatible with the tissue of the cornea. An illustrative material is a plastic type material sold under the trade name of PERSPEX CQ™ (Imperial Chemical Company, England), however many other biocompatible polymers including but not limited to Teflon, and polysulfones are useful in the invention. One acceptable cross-sectional shape of the rings is the hexagonal shape shown in Figs.6A and 6B and is generally dimensioned to be about 0.5 mm to 2.0 mm from point to point along its major axis (dimension "x") and from about 0.05 mm to 0.60 mm in thickness (dimension "y").

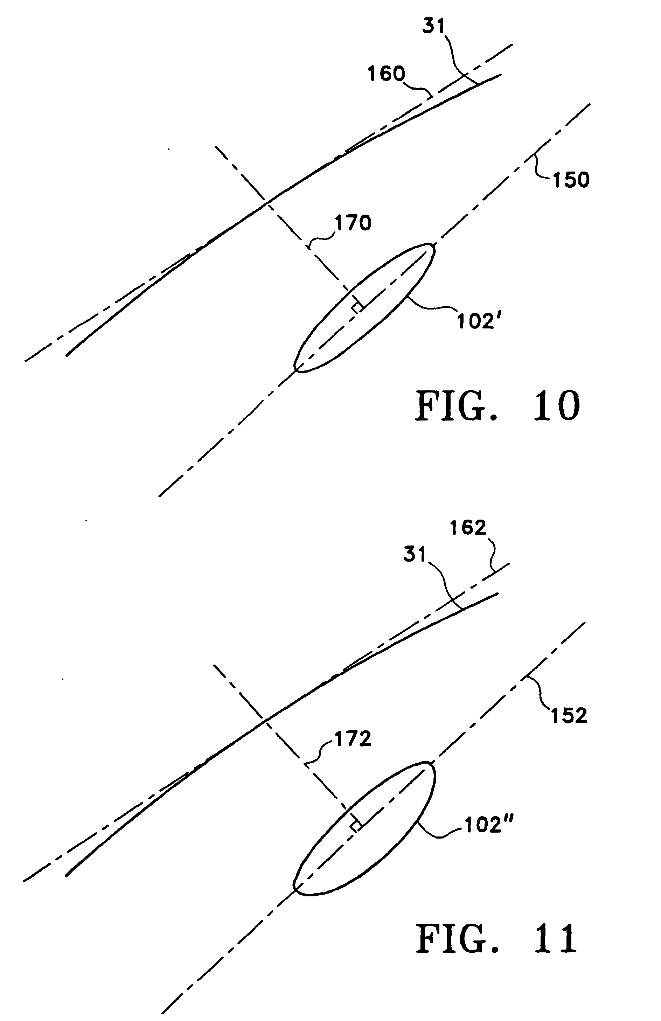

Rings of other cross-sectional shapes including but not limited to ovoloid and rectangular shapes may be useful in the invention as well. Illustrative examples of generally ovoloid shapes are provided in Figs. 10 and 11, which will be discussed in more detail below. It should also be understood that although a split ring is shown, continuous or closed rings can be used as well as other ring configurations.

Referring to Fig. 6B, the cone angle N(θ) of an intrastromal corneal ring is defined, for example, as the angle between the plane of the flat surface 50 that the intrastromal corneal ring rests on and a line drawn between points 52 of the cross-section that rests on the flat surface and point 54 on the cross-section that is farthest from the point where the intrastromal conical ring rests on the flat surface. In other words, cone angle is the angle formed between the major axis of a radial, transverse cross-section (e.g., axis 56 as shown in Fig. 6B) and surface 50. Alternatively, the cone angle can be defined with reference to the angle formed by the intersection of major axes 56, angle φ , where $N(\theta) = (180-\phi)/2$ .

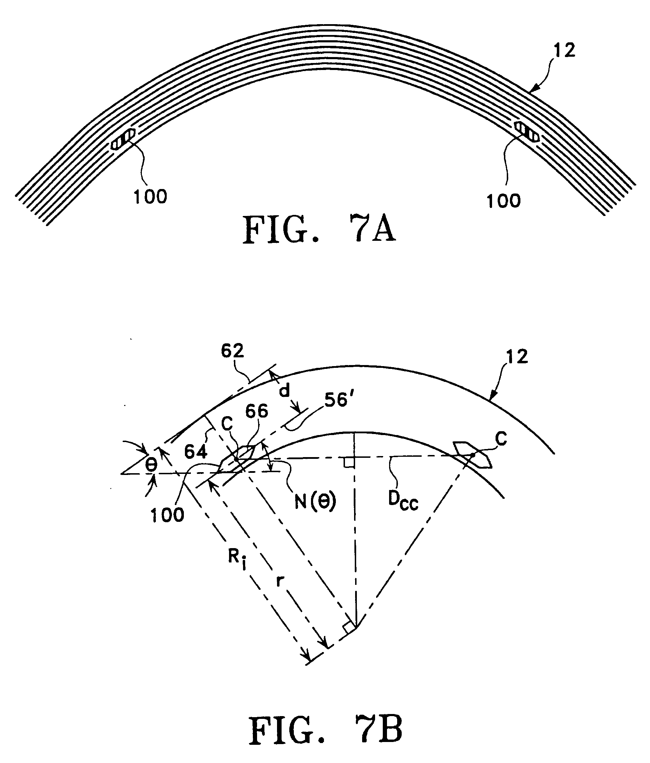

An important aspect of the invention is that the shape of the anterior conical surface may be adjusted by using intrastromal conical rings having mismatching "cone angles". This is generally illustrated in Figs. 7-9 where the effect of mismatching and matching cone angles is compared. Referring to Fig. 7A, the effect of inserting an intrastromal corneal ring, such as intrastromal corneal ring 100 discussed above, having a cone angle matched to the corneal architecture prior to insertion is generally shown.

A matched cone angle may generally be considered to be one that matches the angle of a line that is tangent to the anterior surface of the cornea. That tangent line is obtained by radially projecting the line that extends along the major axis of a transverse cross-section of the intrastromal corneal ring (e.g., along the line indicating dimension x in Figs. 6A and 6B) to the corneal anterior surface as shown in Fig. 7A. A matching cone angle may vary according to changes in corneal shape and to the dimension of the intrastromal corneal ring used.

More specifically, a matching cone angle can be described as follows with reference to an imaginary intrastromal corneal ring superimposed at the insertion site prior to insertion of the intrastromal corneal ring. The major axis of substantially any transverse cross-section of the intrastromal corneal ring would be parallel to a line in the same plane as said axis and tangent to the anterior surface of the cornea at the point where the line that bisects said major axis line (defined as the line extending along said major axis and bounded by the outer surface of the intrastromal corneal ring, and is perpendicular thereto) intersects the anterior surface of the cornea.

In deriving an equation to calculate matching cone angles, applicants considered the variables listed below to be important in determining the corneal radius change. $\Delta R_{0s}$, achieved by implanting the ring in the cornea.

$$\Delta R_{0s} = f(R_i, \theta, t, d, D_i, D_{cc}, E_y, O_s) \text{ where}$$

$R_i$ = initial corneal radius of curvature (measured along the anterior corneal surface)

θ = cone angle of the intrastromal corneal ring

t = intrastromal corneal ring thickness

$D_i$ = limbus diameter

$D_{cc}$ = diameter of intrastromal corneal ring (c-c)

$E_y$ = Young's modulus for the intrastromal corneal ring

$0_s$ = intrastromal corneal ring cross-sectional area shape factor

$$= f\left(\frac{LXA}{CXA}\right)$$

where LXA is the long axis of the radial, transverse cross-sectional area of the intrastromal corneal ring and CXA is the circumference of the radial, transverse cross-sectional area of the intrastromal corneal ring.

These variables are believed to be the dominant ones that will indicate how the intrastromal corneal ring will perform in changing corneal curvature or shape.

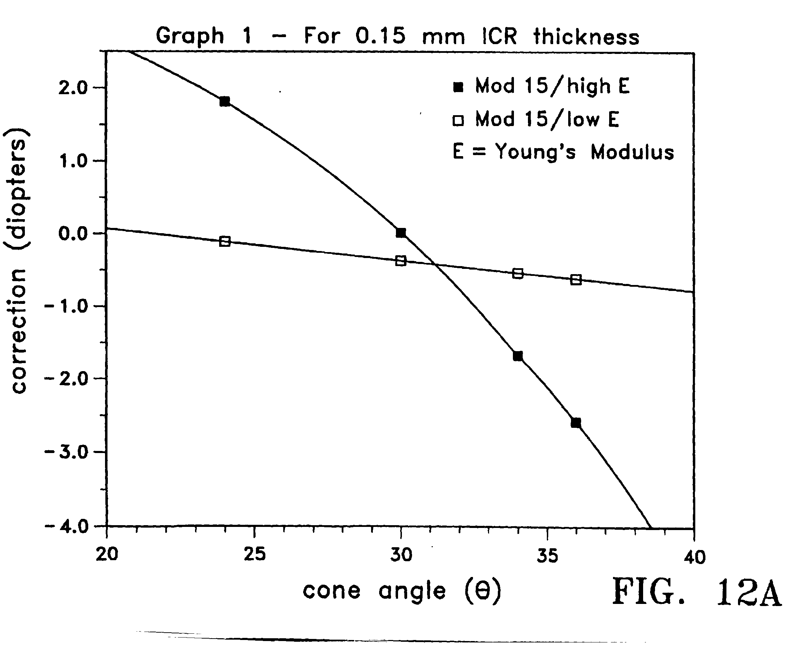

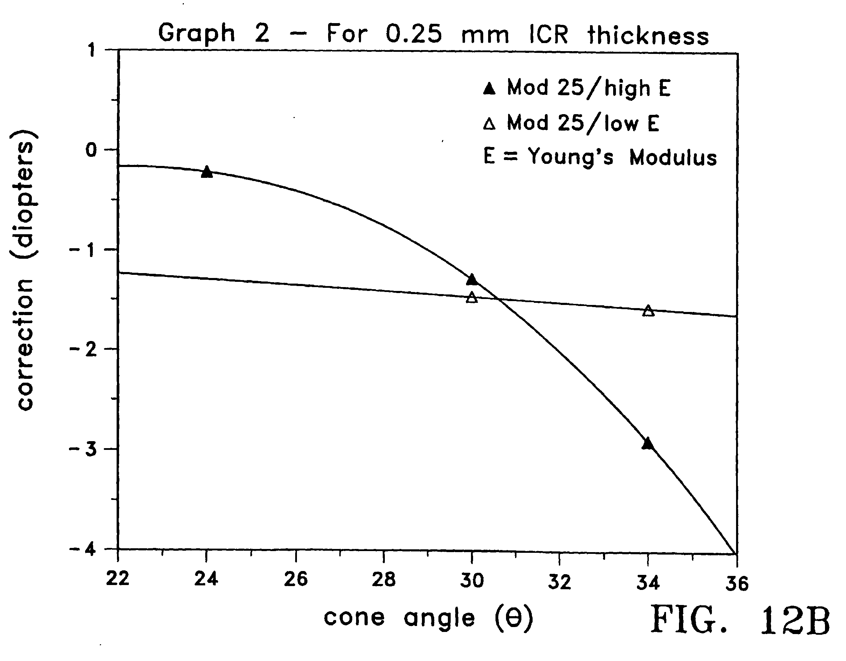

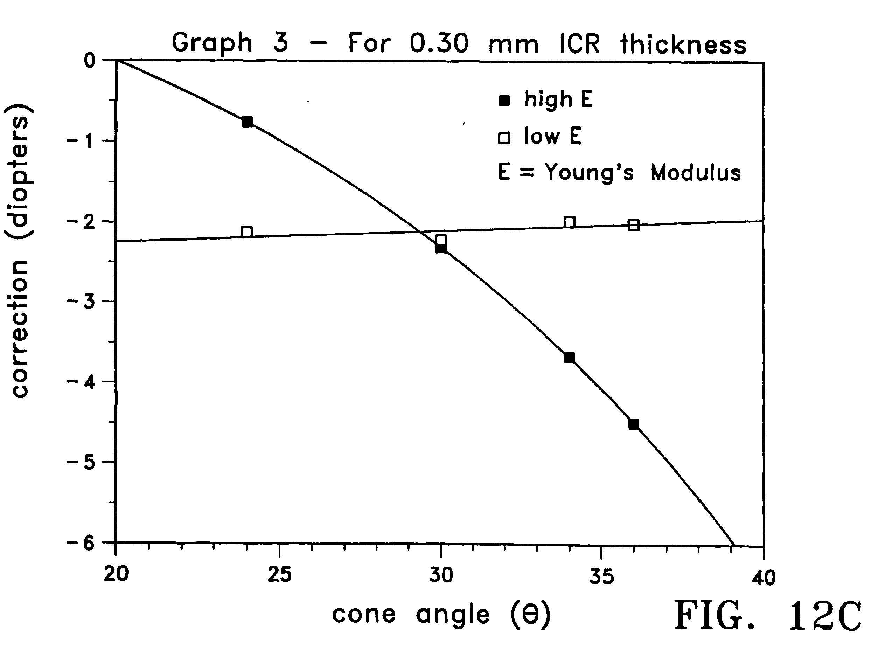

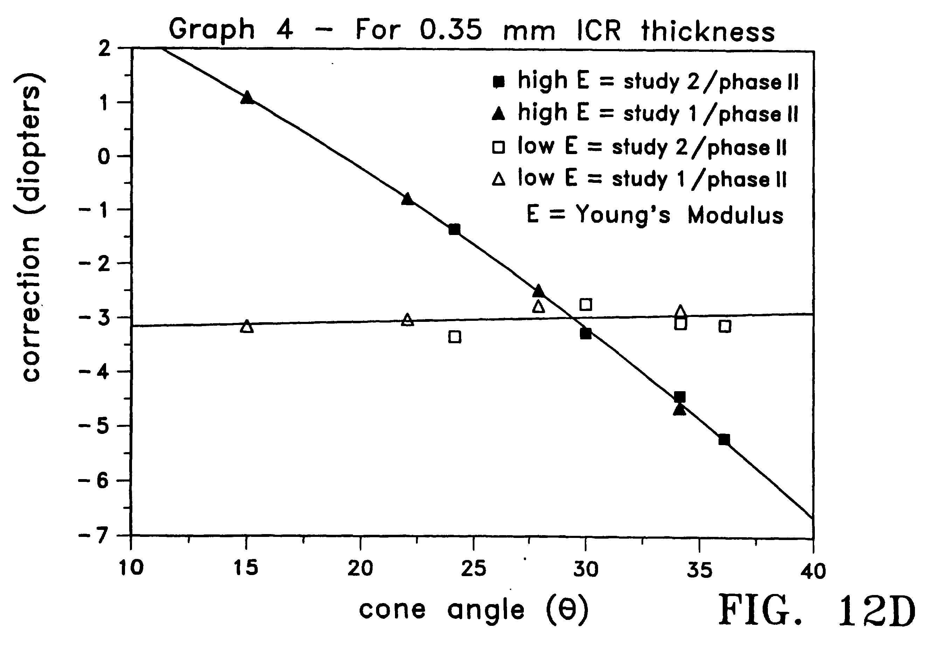

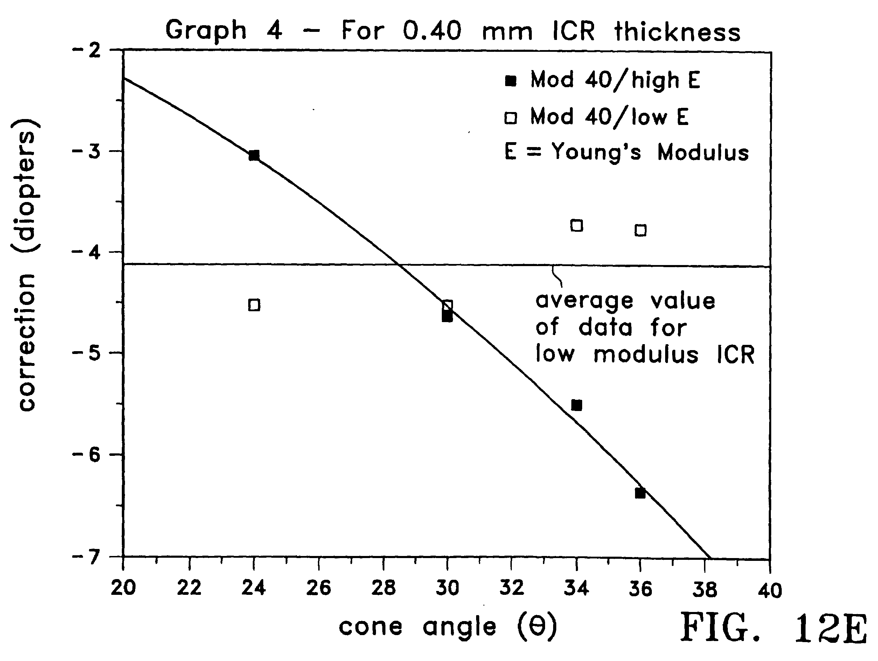

The graphs shown in Figs. 12A, 12B, 12C, 12D and 12E show an example of fixing every variable constant with the exception of cone angle and Young's modulus at a given thickness. The point where the curves intersect generally corresponds to a matched cone angle. In general, with a high modulus, an increase in cone angle provides an increase in the minus correction which means an increase in the flattening of the cornea. By lowering the cone angle, one induces a positive correction which results in steepening the cornea. Correction is proportional to the radial curvature of the cornea. The correction values in the graphs are 337.5/(the initial radius of corneal curvature - the final radius of corneal curvature).

Further examples indicating that increasing the cone angle increases refractive corrections are provided with respect to data obtained from tests conducted on human eye bank eyes. A cone angle of 25°, 34° and 40° provided refractive corrections of about -1.9, -2.7 and -6.0 diopters, respectively. Although the intrastromal corneal ring cone angles varied, each intrastromal corneal ring had a thickness (t) of about 0.30 mm and was inserted at a depth (d) in the cornea of about 0.42 mm.

Based on the variables listed above, the following equation, which defines a matching cone angle, can be derived for an intrastromal corneal ring of given thickness, cross-sectional shape, Young's modulus and unknown limbal diameter.

$$\theta = \sin^{-1}\left[\frac{D_{cc}}{2R_i}\right], \qquad\qquad \text{Equation (1)}$$

where

θ = cone angle

$D_{cc}$ = diameter of the intrastromal corneal ring (center to center)

$R_i$ = initial corneal radius of curvature

Variable "d" (depth of the implant measured from the anterior corneal surface) does not appear in equation (1) because in this simplified version "d" is zero and the radius of curvature corresponds to that at the corneal surface. The following version of equation (1), including dimension d, can alternatively be used as would be readily apparent to one of ordinary skill.

$$\theta = \sin^{-1}\left[\frac{D_{cc}}{2(R_i - d)}\right], \qquad\qquad \text{Equation (2)}$$

where

θ, $D_{cc}$, $R_i$ and d are as defined above. Referring to Fig. 7B. depth (d) can be more specifically defined as the implant position in the cornea measured radially from the anterior corneal surface to the midpoint of a radial line, extending across the thickest or largest radial dimension (e.g., "y" in Fig. 6A) of the radial, transverse section referenced above with respect to $D_{cc}$ and shown, for example, in Fig. 7B. Also illustrated in Fig. 7B is center to center diameter $D_{cc}$ where each center is at the midpoint of the major axis line of a radial, transverse section of the intrastromal corneal ring. Such centers are shown in Fig. 7B, for example, where they are indicated with reference character "c".

The following table provides matching cone angle values ($\theta$) in degrees rounded to the nearest tenth of a degree for an intrastromal comeal ring implanted at a depth of 0.42 mm and for an initial comeal radius of curvature ($R_i$) ranging from 7.2-8.1 mm (which is the typical range in the population) and center to center diameters ($D_{cc}$) ranging from 5.0-8.0 mm according to the above equation (2).

| | $D_{cc}$ | | | | | | |
|---|---|---|---|---|---|---|---|
| $R_i$ | 5.0 | 5.5 | 6.0 | 6.5 | 7.0 | 7.5 | 8.0 |
| 7.2 | 21.6 | 23.9 | 26.3 | 28.6 | 31.1 | 33.6 | 36.2 |
| 7.5 | 20.7 | 22.9 | 25.1 | 27.3 | 29.6 | 32.0 | 34.4 |
| 7.6 | 20.4 | 22.5 | 24.7 | 26.9 | 29.2 | 31.5 | 33.9 |
| 7.7 | 20.1 | 22.2 | 24.3 | 26.5 | 28.7 | 31.0 | 33.3 |
| 7.8 | 19.8 | 21.9 | 24.0 | 26.1 | 28.3 | 30.5 | 32.8 |
| 7.9 | 19.5 | 21.6 | 23.7 | 25.8 | 27.9 | 30.1 | 32.3 |
| 8.1 | 19.0 | 21.0 | 23.0 | 25.0 | 27.1 | 29.2 | 31.4 |

The above calculations are merely exemplary and not intended to limit the invention. For example, the implantation depth "d" may range from about 0.10-0.50 mm, even though a value of 0.42 mm is used throughout the above calculations for purposes of example.

A mismatching cone angle is one that does not equal the cone angle described in equation (1) or (2) for a given $D_{cc}$, $R_i$ and d. Thus, for example, given a $D_{cc}$ of 7.0 mm, an $R_i$ of 7.7 mm and a "d" of 0.42 mm, a mismatching cone angle would be any cone angle that is not equal to 28.7 degrees.

One method to practice the invention is for a clinician to have a kit of rings containing a number of different cone angles to choose from. Typically, a kit having multiple rings, preferably with the same $D_{cc}$, but different cone angles will be provided for clinical use. These rings will typically vary from a matched cone angle for a given $R_i$ by at least 1°, more preferably by at least 2°, even more preferably by at least 3° and yet more preferably by at least 5° (to facilitate substantial corrections as shown in Figs. 12A-E) so that if one does not provide the desired correction, another one can be used (i.e., if the first ring selected does not provide the desired correction, it can be replaced by another one from the kit). Preferably, the kit will include a ring having a cone angle greater than and a ring having a cone angle less than that of a matched cone angle for a given $R_i$ by the foregoing amounts to facilitate flattening or steepening of the cornea as discussed above. Thus, in the foregoing example where $D_{cc}$ is 7.0 mm and a matching cone angle is 28.7°, the kit may include a plurality of rings having a $D_{cc}$ of 7.0 mm with at least one having a cone angle at least 3° more than 28.7° and another having a cone angle at least 3° less than 28.7°. The kit also may include other rings, including one having a matching cone angle. All or a lesser number of the rings also may vary in 0.5° (or larger) increments from one another and have the same thickness. With a kit having rings all with the same $D_{cc}$ or ring diameter, selection of the properly mismatching ring may be simplified. This feature also allows the kit to be made as small as possible, while covering as large a range of correction as possible.

Another equation may be preferred when accounting for thicknesses (t) above about 0.15 mm, which provides sufficient thickness to flatten the cornea independent of cone angle. According to this refined equation:

$$\theta = \sin^{-1}\left[\frac{D_{cc}}{2(R_i - \Delta R_t)}\right] \qquad \text{Equation (3)}$$

Another way to express equation 3 is

$$\theta = \sin^{-1}\left[\frac{D_{cc}}{2(R_i+|\Delta R_t|)}\right], \text{ where}$$

$\Delta R_i$ = the expected radius of corneal curvature change induced by intrastromal corneal ring thickness independent of cone angle for a given transverse cross-sectional shape of any particular intrastromal corneal ring design where, as apparent from the above, the change is measured as the initial radius of corneal curvature (i.e., the radius of curvature of the cornea prior to intrastromal corneal ring implantation) minus the final radius of corneal curvature (i.e., the radius of curvature of the cornea after intrastromal corneal ring implantation).

Again θ can be defined using an equation accounting for the actual implantation depth of the intrastromal corneal ring. According to this refined equation (4):

$$\theta = \sin^{-1}\left[\frac{D_{cc}}{2[Ri-d)+|\Delta R_t|]}\right] \qquad \text{Equation (4)}$$

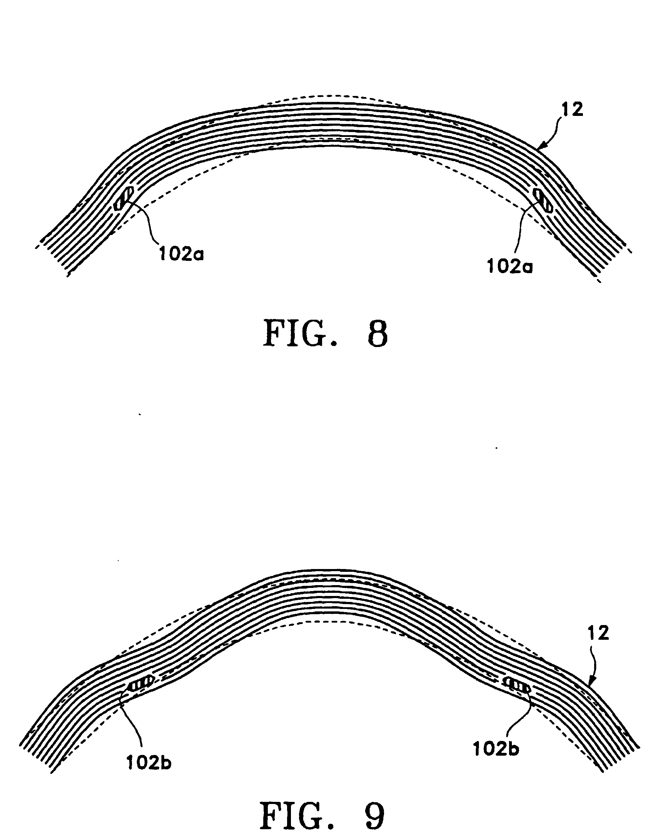

Returning to Figs. 8 and 9, where intrastromal corneal rings having mismatched cone angles are shown, further principles of the invention will be described. Referring to Fig. 8, intrastromal corneal ring 102a is shown with a core angle greater than that shown in Fig. 7. This twists adjacent portions of the cornea outward and flattens the central region of the cornea within the intrastromal corneal ring diameter as shown in the drawing. In contrast, Fig. 9 shows an intrastromal corneal ring 102b, which has a cone angle less than that shown in Fig. 7A, according to another emodiment of the invention. This cone angle effects a steepening of the corneal surface as shown in the drawing. In Figs. 8 and 9 the phantom lines correspond to the outer lines of the cornea section of Fig. 7A and thus provide a reference for the changes in Figs. 8 and 9.

Referring to Figs 10 and 11, the concept of a mismatching cone angle will be described again with reference to an imaginary intrastromal corneal ring (102', 102") superimposed at the insertion site prior to insertion of the cornea. The major axis (axes 150 and 152 in Figs. 10 and 11, respectively) of substantially any radial, transverse cross-section of the intrastromal corneal ring would not be parallel to a line (lines 160 and 162 in Figs. 10 and 11, respectively), which in the same plane as said major axis and is tangent to the anterior surface of die cornea at the point where the line (line 170 and 172 in Figs. 10 and 11, respectfully) that bisects said major axis line (defined as the line extending along said major axis and bounded by the outer surface of the intrastromal corneal ring) and is perpendicular thereto, intersects the anterior surface of the cornea. Alternatively, a mismatching cone angle may be defined as one that is outside one of the equations described above.

The intrastromal corneal ring may be installed in the inner lamellar regions of the corneal stroma by any of the methods we have shown in the past to be suitable for such installation. Particularly desired is the process and its allied apparatus shown in PCT/US93/03214 which is incorporated herein by reference in its entirety. In general, the ring is installed in the foregoing manner: A small radial incision is made at the radius in which the ring is ultimately to be installed about the cornea. A dissector in the form of a split ring and having a point suitable for producing an interlamellar channel or tunnel in the corneal stroma is introduced through the small incision and rotated in such a fashion that a generally circular channel is formed completely about the cornea. The dissector is then rotated in the opposite direction to withdraw it from the tunnel thus formed. The intrastromal corneal ring is then introduced into the circular channel. Alternatively, a clockwise and counterclockwise channel dissection method can be used using a clockwise and counterclockwise dissector system as disclosed in PCT/US95/00063 entitled System For Inserting Material Into Corneal Stroma, which is hereby incorporated herein in its entirety. The system generally includes a clockwise and counterclockwise channel dissector; a clockwise and counterclockwise probe for inserting in the channels and determining the relative positions of the channel ends (e.g., if the channels meet); a clockwise and counterclockwise channel connector that may subtend an arc of about 240° to 360° to connect the channels if they do not meet (by rotation of the connector in the lower channel); and a clockwise and counterclockwise finish channel connector thay may subtend an arc of about 360° to 510° for insertion into the lower channel and connect the channels if the channel connector does not accomplish that effect.

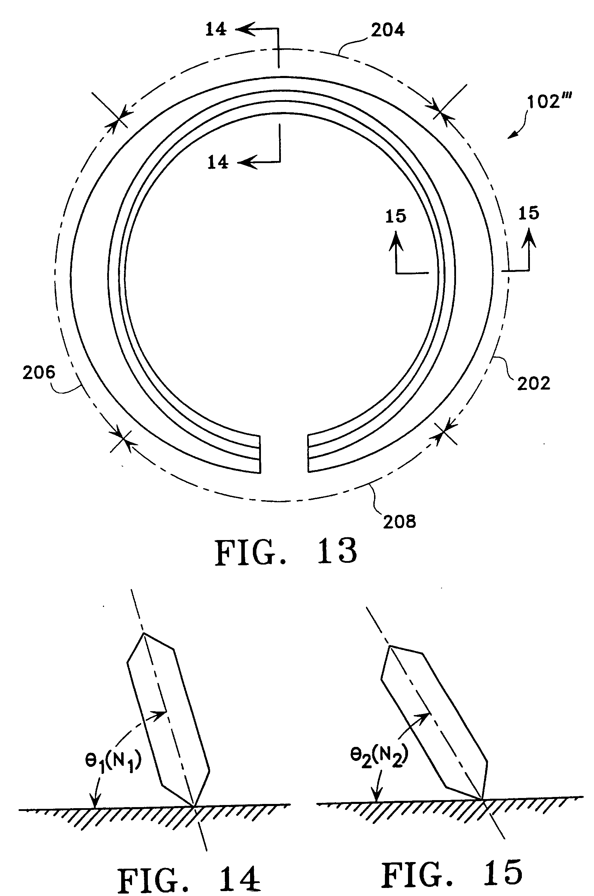

Referring to Figs. 13-15, a further embodiment of the invention is shown. In this embodiment, intrastromal corneal ring 102''', which preferably is constructed of materials as described above with respect to the example shown in Figs. 5 and 6, is provided with multiple cone angles.

That is, the intrastromal corneal ring cone angle changes along the circumferential direction thereof as shown in Fig 13. Generally speaking, the intrastromal corneal ring has a first circumferential region having a first cone angle and at least one other region having a cone angle that differs form said first cone angle.

In the example illustrated in Fig. 13, the intrastromal corneal ring has four circumferential regions with distinct cone angles. Circumferential region 202 has a first cone angle as shown in Fig.15. Circumferential region 202 is followed by a second circumferential region 204 having a second cone angle (Fig. 14) that substantially differs from the first cone angle. Circumferential region 206 follows with a cone angle similar to that of region 202 and region 208 has a cone angle similar to region 204. The intrastromal corneal ring preferably is configured to substantially encircle the cornea after insertion. However, although a split ring configuration is shown, continuous or closed loop as well as other ring configurations can be used. The planes of Figs. 14 and 15 are parallel but may or may not be in the same plane depending on the ring design. A saddle shaped ring would not have its base lie in one plane, for example.

This embodiment is particularly advantageous for treating astigmatism (or astigmastism concurrent with either myopia or hyperopia as discussed in the summary of the invention, *supra*), which is a condition which occurs when the parallel rays of light do not focus to a single point within the eye, but rather have a variable focus due to the fact that the comeal curvature varies in different meridians. Accordingly, the cone angle varies enough for treating this indication. This variation typically may range from about 0° 10' to 20°, and more typically from about 3° to 20°. A region having one cone angle typically may subtend an arc more than at least 2°. The cone angle transitions between regions may be abrupt or gradual depending on the desired change to the corneal shape. In addition, a saddle shaped ring may be used as well as rings having more circumferential regions or circumferential regions having different shapes or dimensions than those shown. Rings having their centroid axis in one plane or their centroidal axis following a saddle shape also are contemplated.

Modifications of the above described modes for carrying out the invention that are obvious to persons of skill in the fields of medicine, ophthalmology, optometry and/or related fields are intended to be within the scope of the following claims. The claims and specification should not be construed to unduly narrow the full scope of protection to which the invention is entitled.

**Claims**

1. An intrastromal corneal ring for insertion into the cornea of an eye, said intrastromal ring comprising a biocompatible ring having multiple cone angles.

2. The intrastromal ring of claim 1, wherein the cone angle of the intrastromal ring changes along the circumferential direction of the ring.

3. The intrastromal ring of claim 1, wherein the intrastromal ring has a first circumferential region having a first cone angle and at least one other circumferential region positioned away from said first circumferential region in a circumferential direction and having a cone angle that differs from said first cone angle.

4. The intrastromal ring of claim 1, wherein the intrastromal ring has a split ring configuration.

5. The intrastromal ring of claim 1, wherein said angles sufficiently differ for treating astigmatism in a human eye.

6. The intrastromal ring of claim 5, wherein said angles differ from one another by about 0° 10' to about 20°.

7. The intrastromal ring of claim 6, wherein said angles differ from another by about 3° to 20°.

8. The intrastromal ring of claim 1, wherein said ring has at least four regions positioned along the ring in a circumferential direction, adjacent regions having different cone angles.

FIG. 1

FIG 2

FIG. 3

FIG. 4

FIG. 5

6A     6A          6A     6A

102

FIG. 6A

N(θ)

y
x

FIG. 6B

56          Φ          56

54

y
x

θ(N)          θ

52          50

## FIG. 7A

## FIG. 7B

12

102a

102a

FIG. 8

12

102b

102b

FIG. 9

FIG. 10

FIG. 11

Graph 1 — For 0.15 mm ICR thickness

FIG. 12A

EP 1 060 715 A1

Graph 2 — For 0.25 mm ICR thickness

FIG. 12B

EP 1 060 715 A1

Graph 3 — For 0.30 mm ICR thickness

- high E
- low E

E = Young's Modulus

correction (diopters)

cone angle (Θ)

FIG. 12C

EP 1 060 715 A1

Graph 4 — For 0.35 mm ICR thickness

■ high E = study 2/phase II
▲ high E = study 1/phase II
□ low E = study 2/phase II
△ low E = study 1/phase II
E = Young's Modulus

correction (diopters)

cone angle (θ)

FIG. 12D

EP 1 060 715 A1

Graph 4 — For 0.40 mm ICR thickness

FIG. 12E

EP 1 060 715 A1

FIG. 13

FIG. 14

FIG. 15

**European Patent Office**

**EUROPEAN SEARCH REPORT**

Application Number

EP 00 11 5411

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 93 12735 A (CHIRON) 8 July 1993 (1993-07-08) * page 6, line 4 - page 7, line 23; figure 2 * | 1 | A61F2/14 |
| X | SU 388 746 A (MOSCOW HELMHOLTZ EYE CLI) 5 July 1973 (1973-07-05) * figure 1 * | 1 | |
| A,D | WO 93 13724 A (KERAVISION) 22 July 1993 (1993-07-22) * page 8, line 8 - line 26 * | 1 | |
| A | WO 94 05232 A (KERAVISION) 17 March 1994 (1994-03-17) * abstract; figures 3,4 * | 1 | |
| A | WO 95 03755 A (KERAVISION) 9 February 1995 (1995-02-09) * page 9, line 25 - page 10, line 2 * * page 17, line 15 - page 18, line 7; figures 3B,10-12 * | 1 | TECHNICAL FIELDS SEARCHED (Int.Cl.7) |
| A | WO 94 06504 A (KERAVISION) 31 March 1994 (1994-03-31) * page 13, line 3 - page 14, line 18; figure 6B * | 1 | A61F |
| A,P | WO 95 05789 A (KERAVISION) 2 March 1995 (1995-03-02) * abstract; figure 6 * | 1 | |

The present search report has been drawn up for all claims

| Place of search | Date of completion of the search | Examiner |
|---|---|---|
| THE HAGUE | 15 August 2000 | Moers, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 00 11 5411

This annex lists the patent family members relating to the patent documents cited in the above-mentioned European search report.
The members are as contained in the European Patent Office EDP file on
The European Patent Office is in no way liable for these particulars which are merely given for the purpose of information.

15-08-2000

| Patent document cited in search report | | Publication date | Patent family member(s) | | Publication date |
|---|---|---|---|---|---|
| WO 9312735 | A | 08-07-1993 | AT | 189591 T | 15-02-2000 |
| | | | AU | 3417793 A | 28-07-1993 |
| | | | CA | 2127109 A | 08-07-1993 |
| | | | DE | 69230673 D | 16-03-2000 |
| | | | DE | 69230673 T | 29-06-2000 |
| | | | EP | 0619724 A | 19-10-1994 |
| | | | ES | 2143501 T | 16-05-2000 |
| | | | JP | 7506730 T | 27-07-1995 |
| | | | US | 5391201 A | 21-02-1995 |
| SU 388746 | A | 05-07-1973 | NONE | | |
| WO 9313724 | A | 22-07-1993 | AU | 3433693 A | 03-08-1993 |
| | | | BR | 9305734 A | 28-01-1997 |
| | | | CN | 1082390 A | 23-02-1994 |
| | | | EP | 0621763 A | 02-11-1994 |
| | | | JP | 7506014 T | 06-07-1995 |
| | | | SG | 49160 A | 18-05-1998 |
| | | | US | 5318047 A | 07-06-1994 |
| | | | US | RE35974 E | 01-12-1998 |
| WO 9405232 | A | 17-03-1994 | AT | 183067 T | 15-08-1999 |
| | | | AU | 4847793 A | 29-03-1994 |
| | | | BR | 9307004 A | 23-02-1999 |
| | | | CA | 2143682 A | 17-03-1994 |
| | | | CN | 1095259 A | 23-11-1994 |
| | | | DE | 69326024 D | 16-09-1999 |
| | | | DE | 69326024 T | 24-02-2000 |
| | | | EP | 0686018 A | 13-12-1995 |
| | | | ES | 2134268 T | 01-10-1999 |
| | | | JP | 8501009 T | 06-02-1996 |
| | | | SG | 52740 A | 28-09-1998 |
| | | | US | 5405384 A | 11-04-1995 |
| | | | US | 5944752 A | 31-08-1999 |
| WO 9503755 | A | 09-02-1995 | AU | 6996398 A | 16-07-1998 |
| | | | AU | 687859 B | 05-03-1998 |
| | | | AU | 7515694 A | 28-02-1995 |
| | | | BR | 9407215 A | 17-09-1996 |
| | | | CA | 2168347 A | 09-02-1995 |
| | | | EP | 0712301 A | 22-05-1996 |
| | | | IL | 110532 A | 06-12-1998 |
| | | | JP | 9503930 T | 22-04-1997 |
| | | | SG | 52643 A | 28-09-1998 |
| | | | US | 5824086 A | 20-10-1998 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**                    EP 00 11 5411

This annex lists the patent family members relating to the patent documents cited in the above-mentioned European search report.
The members are as contained in the European Patent Office EDP file on
The European Patent Office is in no way liable for these particulars which are merely given for the purpose of information.

15-08-2000

| Patent document cited in search report | | Publication date | Patent family member(s) | | Publication date |
|---|---|---|---|---|---|
| WO 9406504 | A | 31-03-1994 | US | 5300118 A | 05-04-1994 |
| | | | AT | 183106 T | 15-08-1999 |
| | | | AU | 684723 B | 08-01-1998 |
| | | | AU | 4926293 A | 12-04-1994 |
| | | | BR | 9307076 A | 29-06-1999 |
| | | | CA | 2144761 A | 31-03-1994 |
| | | | CN | 1091000 A | 24-08-1994 |
| | | | DE | 69326011 D | 16-09-1999 |
| | | | DE | 69326011 T | 24-02-2000 |
| | | | EP | 0660732 A | 05-07-1995 |
| | | | ES | 2134270 T | 01-10-1999 |
| | | | IL | 107037 A | 15-06-1998 |
| | | | JP | 8504114 T | 07-05-1996 |
| | | | US | 5466260 A | 14-11-1995 |
| | | | US | 5693092 A | 02-12-1997 |
| WO 9505789 | A | 02-03-1995 | AU | 7673494 A | 21-03-1995 |
| | | | BR | 9407359 A | 29-10-1996 |
| | | | EP | 0746272 A | 11-12-1996 |
| | | | IL | 110735 A | 05-04-1998 |
| | | | JP | 9504448 T | 06-05-1997 |
| | | | SG | 48137 A | 17-04-1998 |

EPO FORM P0459

For more details about this annex : see Official Journal of the European Patent Office, No. 12/82